# EUROPEAN PATENT SPECIFICATION

(11) **EP 2 254 612 B1**
(45) Date of publication and mention of the grant of the patent: **09.10.2019**
(21) Application number: 09722748.2
(22) Date of filing: 20.03.2009
(51) Int. Cl.: A61M 1/00

(54) **WOUND THERAPY SYSTEM**
WUNDTHERAPIESYSTEM
SYSTÈME DE THÉRAPIE DE PLAIE

(30) Priority: 09.05.2008 US 52007 P; 12.06.2008 US 60869 P; 20.03.2008 US 38301 P; 21.01.2009 US 146051 P
(43) Date of publication of application: 01.12.2010
(73) Proprietor: Smith & Nephew, Inc., Memphis, TN 38116 (US)
(72) Inventor: BRAGA, Richard, M., North Easton, MA 02356 (US); VITARIS, Ronald, F., Worcester, MA 01606 (US); COOKE, Shane, Wrentham, MA 02093 (US); HEAGLE, David, G., Taunton, MA 02780 (US); HUDSPETH, Michael, D., Arnold, MA 63010 (US); MALHI, Arnaz, Watertown, MA 02472 (US); SWANIKER, Hansen, Bristol, RI 02809 (US); WATSON, Kristin, L., North Attleboro, MA 02760 (US)
(74) Representative: Smith & Nephew
(86) International application number: PCT/US2009/037762
(87) International publication number: WO 2009/117635

(56) References cited:
- WO-A1-2007/087808
- WO-A1-2007/087809
- WO-A2-03/101508
- GB-A- 1 415 096
- US-A- 3 980 166
- US-A1- 2007 032 762
- US-A1- 2007 167 927
- US-A1- 2007 167 927
- US-A1- 2007 179 460

## Description

### BACKGROUND

### 1. Technical Field

The present disclosure relates to treating an open wound, and, more specifically, relates to a portable wound therapy system.

### 2. Description of Related Art

Wound closure involves the migration of epithelial and subcutaneous tissue adjacent the wound towards the center and away from the base of the wound until the wound closes. Unfortunately, closure is difficult with large wounds, chronic wounds or wounds that have become infected. In such wounds, a zone of stasis (i.e. an area in which localized swelling of tissue restricts the flow of blood to the tissues) forms near the surface of the wound. Without sufficient blood flow, the epithelial and subcutaneous tissues surrounding the wound not only receive diminished oxygen and nutrients, but, are also less able to successfully fight microbial infection and, thus, are less able to close the wound naturally. Such wounds have presented difficulties to medical personnel for many years.

Negative pressure therapy also known as suction or vacuum therapy has been used in treating and healing wounds. Application of negative pressure, e.g. reduced or subatmospheric pressure, to a localized reservoir over a wound has been found to assist in closing the wound by promoting blood flow to the area, stimulating the formation of granulation tissue, and encouraging the migration of healthy tissue over the wound. Negative pressure may also inhibit bacterial growth by drawing fluids from the wound such as exudates, which may tend to harbor bacteria. This technique has proven particularly effective for chronic or healing-resistant wounds, and is also used for other purposes such as postoperative wound care.

Generally, negative pressure therapy provides for a wound to be covered to facilitate suction at the wound area. A conduit is introduced through the wound covering to provide fluid communication to an external vacuum source. Atmospheric gas, wound exudates, or other fluids may thus be drawn from the reservoir through the fluid conduit to stimulate healing of the wound. Exudates drawn from the reservoir may be deposited in a collection canister. The various components of the wound therapy system may need to be disconnected or be replaced for a variety of reasons, such as component failure or different component life expectancies. It would be advantageous to provide a user friendly mechanism for connecting and disconnecting components of the system.

Patent Application documents US2007/167927, US2007/032762, WO2007/087809, WO2007/087808, US2007/179460 and WO03/101508 all disclose prior art negative pressure therapy systems.

### SUMMARY

Accordingly, the present invention relates to a portable system for subatmospheric pressure therapy in connection with healing a surgical wound, as defined by the appended claims. The system includes a wound dressing dimensioned for positioning relative to a wound bed of a subject and a portable subatmospheric pressure mechanism dimensioned to be carried or worn by the subject. The subatmospheric pressure mechanism includes a housing having a control unit, a collection canister, and means for releasably connecting the housing and the canister.

Various means for releasably coupling the housing and the canister are disclosed herein, including latch couplings, bayonet mounts, fastening mechanisms, and magnetic couplings. According to the invention as claimed, the means for releasably coupling the housing and the canister includes a latch coupling mechanism that incorporates an elongate latch defining a general "z-shape" and comprises a locking surface positioned substantially at the center of the latch. The connection means may facilitate the connection, disconnection, or maintenance of components of the system including the replacement of the collection canister. The connection means permits the collection canister to be released for emptying or disposal during a course of therapy. In one method of application, the collection canister collects exudates from the wound bed which has been removed under subatmospheric pressure supplied by the control unit. When full, the collection canister is removed and replaced with a new canister which is also connectable through connection means with the housing of the control unit. The control unit may be intended for reuse.

### BRIEF DESCRIPTION OF THE DRAWINGS

Various embodiments of the wound dressing system of the present disclosure are described herein with reference to the drawings wherein:
**FIG. 1A** is a view of the portable wound therapy system of the present disclosure illustrating the wound dressing in cross-section and the subatmospheric pressure mechanism;
**FIG. 1B** is a cross-sectional view of a subatmospheric pressure mechanism in accordance with the present disclosure;
**FIG. 2A** is a perspective view of the subatmospheric pressure mechanism illustrating the control unit housing, collection canister and a latch coupling for releasably connecting the collection canister to the control unit housing;
**FIG. 2B** is a side plan view of the latch coupling of **FIG. 2A**;
**FIG. 2C** is a perspective view of an embodiment of a latch coupling mechanism for releasably connecting the control unit housing and the collection canister;
**FIG. 2D** is a side plan view of the latch coupling mechanism of **FIG. 2C**;
**FIG. 3A** is a perspective view of an example of a latch coupling mechanism for releasably connecting the control unit housing and the collection canister;
**FIG. 3B** is a perspective view of an example of a latch coupling mechanism of **FIG. 3A**;
**FIGS. 4A-4D** are perspective views of other alternative examples of a latch coupling mechanism for releasably connecting the control unit housing and the collection canister;
**FIG. 5** is a perspective view of the collection canister including an example of a second coupling mechanism utilizing a tongue;
**FIG. 6A** is a cross-sectional view of an example of a latch coupling mechanism for releasably connecting the control unit housing and the collection canister;
**FIG. 6B** is a cross-sectional view of the latch coupling mechanism of **FIG. 6A** in a disengaged position;
**FIG. 7A** is a cross-sectional view of an example of a latch coupling mechanism for releasably coupling the control unit housing and the collection canister and depicted in an engaged position;
**FIG. 7B** is a cross-sectional view of the latch coupling of **FIG. 7A** in a disengaged position;
**FIG. 8A** is a view of a carrier support apparatus for supporting components of the subatmospheric pressure mechanism;
**FIG. 8B** is a view of an alternate carrier support apparatus for supporting components of the subatmospheric pressure mechanism of the present disclosure;
**FIG. 9** is a perspective view illustrating an example of a bayonet mount for releasably mounting the control unit housing and the collection canister of the subatmospheric pressure mechanism;
**FIGS. 10A-10C** are partial cross-sectional views illustrating a sequence of operation for connecting the collection canister and the control unit housing with the bayonet mount of **FIG. 9**;
**FIG. 11** is a perspective view illustrating an example of a bayonet mount for releasably mounting the control unit control unit housing and the collection canister of the subatmospheric pressure mechanism;
**FIGS. 12A-12C** are partial cross-sectional views illustrating a sequence of operation for connecting the collection canister and the control unit housing with the bayonet mount of **FIG. 11****;**
**FIGS. 13-15B** are views of different examples of the subatmospheric pressure mechanism having strap fasteners;
**FIGS. 16A-21B** are views of different examples of the subatmospheric pressure mechanism having complementary fastening elements on the periphery of the housing and the canister;
**FIGS. 22A** and **22B** are views of an example of the subatmospheric pressure mechanism having spring-loaded button fasteners in an uncoupled and coupled state, respectively;
**FIGS. 23** and **24** are views of examples of a subatmospheric pressure mechanism having a spring-loaded button fastener;
**FIGS. 25** and **26** are views of different examples of the subatmospheric pressure mechanism having a slide fastener;
**FIGS. 27A-28** are views of examples of a subatmospheric pressure mechanism each having a spring-assisted clip fastener;
**FIGS. 29A** and **29B** are views of an example of the subatmospheric pressure mechanism having movable clip fasteners in an uncoupled and coupled state;
**FIG. 30A** is a cross-sectional view of a magnetic coupling of the control unit and the collection canister of the subatmospheric pressure mechanism;
**FIG. 30B** is a cross-sectional view of an alternate magnetic coupling arrangement of the control unit and collection canister;
**FIG. 30C** is a cross-sectional view of another alternate magnetic coupling arrangement of the control unit and collection canister;
**FIGS. 30D-30F** are top plan views of different magnet configurations within the control unit; and
**FIG. 31** is a cross-sectional view of a magnetic coupling of the control unit and the collection canister via use of permanent magnets.

### DESCRIPTION OF THE EMBODIMENTS

The wound therapy system of the present disclosure promotes healing of a wound via the use of a wound dressing and a subatmospheric pressure mechanism. Generally, the subatmospheric pressure mechanism applies subatmospheric pressure to the wound to effectively remove wound fluids or exudates captured by the composite wound dressing, and to increase blood flow to the wound bed and enhance cellular stimulation of epithelial and subcutaneous tissue. The wound therapy system may be entirely portable, i.e., it may be worn or carried by the subject such that the subject may be completely ambulatory during the therapy period. The wound therapy system including the subatmospheric pressure mechanism and components thereof may be entirely disposable after a predetermined period of use or may be individually disposable whereby some of the components are reused for a subsequent therapy application.

The wound therapy system of the present disclosure promotes healing of a wound in conjunction with subatmospheric negative pressure therapy. The system may incorporate a variety of wound dressings, subatmospheric pressure sources and pumps, and collection canisters.

The attached figures illustrate exemplary embodiments of the present disclosure and are referenced to describe the embodiments depicted therein. Hereinafter, the disclosure will be described by explaining the figures wherein like reference numerals represent like parts throughout the several views.

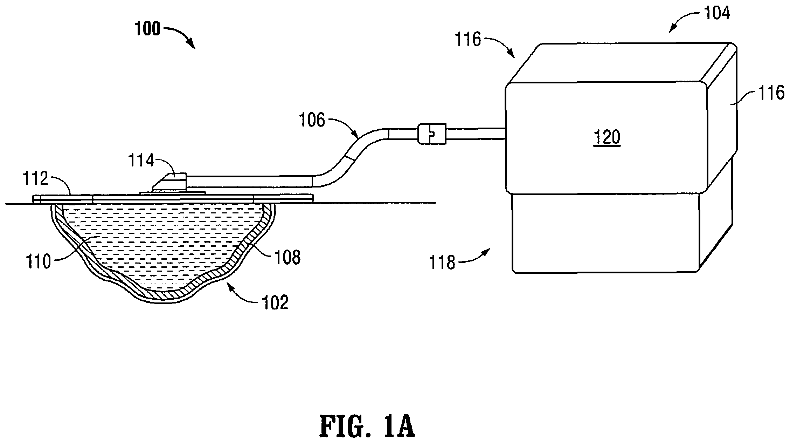

Referring initially to **FIG. 1A**, wound therapy system **100** according to the present disclosure is illustrated. Wound therapy system **100** includes composite wound dressing **102** and subatmospheric pressure mechanism **104** in fluid communication with the wound dressing **102** through conduit **106**.

Wound dressing **102** may include several components, namely, wound contact layer or member **108**, a wound packing member or filler **110** supported by the contact member **108** and outer layer or cover member **112**. Wound contact member **108** is adapted to substantially conform to the topography of a wound bed "w." Wound contact member **108** is substantially porous or perforated to permit exudates to pass from the wound bed "w" through the wound contact member **108**. The passage of wound exudates through the wound contact member **108** may be unidirectional such that wound exudates do not flow back to the wound bed "w." Unidirectional flow may be encouraged by directional apertures formed in contact member **108** or a lamination of materials having absorption properties differing from those of contact member **108.** A non-adherent material may be selected such that contact member **108** does not tend to cling to wound bed "w" or surrounding material when it is removed. Exemplary materials that may be used as a contact member **108** are sold under the trademarks **CURITY® Non-Adherent Dressing** or XEROFLO® by Tyco Healthcare Group, LP (d/b/a Covidien)

Wound packing member **110** of wound dressing **102** is intended to absorb and transfer wound fluid and exudates. Wound packing member **110** is conformable to assume the shape of any wound bed "w." Wound packing member **110** may be treated with agents such as polyhexamethylene biguanide (PHMB) to decrease the incidence of infection, or other medicants to promote healing of the wound. A suitable wound packing material **110** is the antimicrobial dressing sold under the trademark KERLIX™ by Tyco Healthcare Group, LP (d/b/a Covidien).

Outer member or wound covering **112** encompasses the perimeter of the wound dressing **102** to surround wound bed "w" and to provide a liquid-tight seal around the perimeter "p" of the wound bed "w." For instance, the sealing mechanism may be any biocompatible adhesive bonded to the perimeter of wound covering **112.** Thus, wound covering **112** may act as both a microbial barrier and a fluid barrier to prevent contaminants from entering wound bed "w" and for maintaining the integrity thereof.

Wound covering **112** is typically a flexible material, e.g., resilient or elastomeric, that seals the top of wound dressing **102** to prevent passage of liquids or contamination to and from the wound dressing **102**. Wound covering **112** may be formed from a moisture vapor permeable membrane to promote the exchange of oxygen moisture between the wound bed "w" and the atmosphere. A membrane that provides a sufficient moisture vapor transmission rate is a transparent membrane sold under the trade name POLYSKIN® II by Tyco Healthcare Group, LP (d/b/a Covidien). A transparent membrane permits an assessment of wound conditions to be made without requiring removal of the wound covering **112**. Alternatively, wound covering **112** may comprise an impermeable membrane or a substantially rigid membrane.

Wound covering **112** may include a port or connector **114** in fluid communication with the interior of wound dressing **102** to facilitate connection of wound dressing **102** to conduit or tubing **106**. Conduit **106** defines a fluid flow path leading through wound therapy system **100**. Connector **114** may be a rigid or flexible, low-profile component, and may be adapted to receive conduit **106** in a releasable and fluid tight manner. A hollow interior of connector **114** provides fluid communication between conduit **106** and the interior of wound dressing **102**. Connector **114** may have a valve built (not shown), e.g., a one-way valve to permit exudates to flow in one direction only, i.e., away from wound dressing **102** toward subatmospheric pressure mechanism **104**. Connector **114** may be provided as a pre-affixed component of wound dressing **102**, as a component of conduit **106** or entirely separate and connected thereto by conventional means. Alternatively, connector **114** may be eliminated if other provisions are made for providing fluid communication between wound dressing **102** and conduit **106**.

Conduit **106** extends from subatmospheric pressure mechanism **104** to provide fluid communication between the interior of the wound dressing **102** and subatmospheric pressure mechanism **104**. Any suitable conduit may be used including those fabricated from flexible elastomeric or polymeric materials. Conduit **106** may connect to subatmospheric pressure mechanism **104** or other system components by conventional air tight means such as friction fit, bayonet coupling, or barbed connectors. The conduit connections may be made permanent, or alternatively a quick-disconnect or other releasable means may be used to provide some adjustment flexibility to the apparatus. Fluid conduit **106** may comprise the same material of construction along the entire length of the tubing or may assume an alternate form, e.g., it may include several distinct tubes connected to each other through conventional means.

Subatmospheric pressure mechanism **104** will be discussed subatmospheric pressure mechanism **104** includes control unit **116** and collection canister **118**. Control unit **116** has control unit housing **120** which houses the software, logic and components required to operate the subatmospheric pressure mechanism **104**.

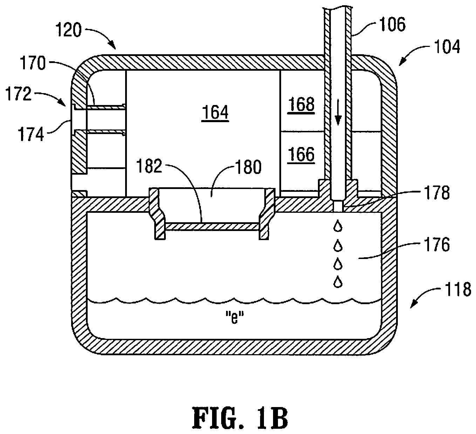

Referring now to **FIG. 1B**, the electronic, electrical and pneumatic components of the subatmospheric pressure mechanism **104** will be discussed. Subatmospheric pressure mechanism **104** may incorporate vacuum source or pump **164**, actuator or motor **166** for activating the vacuum source **164**, and power source **168**. Vacuum source or pump **164** generates or otherwise provides negative pressure to wound therapy system **100**. Vacuum source or pump **164** may be a pump of the diaphragmatic, peristaltic or bellows type or the like, in which the moving part(s) draw exudates out of the wound bed "w" into the wound dressing **102** by creating areas or zones of decreased pressure e.g., vacuum zones with the wound dressing **102**. This area of decreased pressure preferably communicates with the wound bed "w" to facilitate removal of the fluids therefrom and into the absorbent or non-absorbent packing member **110**.

Vacuum source or pump **164** may be a miniature pump or micropump that may be biocompatible and adapted to maintain or draw adequate and therapeutic vacuum levels. The vacuum level of subatmospheric pressure achieved may be in the range of about 20 mmHg to about 500 mmHg. In embodiments, the vacuum level may be about 75 mmHg and about 125 mmHg, or between about 30 mmHg and 80 mmHg. Vacuum source or pump **164** is actuated by actuator **166** which may be any means known by those skilled in the art, including, for example, AC motors, DC motors, voice coil actuators, solenoids, and the like. Actuator **166** may be incorporated within pump **164**.

Power source **168** may be disposed within housing **120** or separately mountable to housing **120**. A suitable power source **168** includes alkaline batteries, wet cell batteries, dry cell batteries, nickel cadmium batteries, solar generated means, lithium batteries, NiMH batteries (nickel metal hydride) each of which may be of the disposable or rechargeable variety.

Housing **120** may further include vent portal **170** configured to vent exhaust air from vacuum source or pump **164** through exhaust port **172**. Vent portal **170** extends from housing **120** and may be directly connected to vacuum source **164**. It is also envisioned that vent portal **170** may exhaust air from within housing **120** rather than directly from vacuum source **164**. Exhaust port **172** may include filter **174** extending across the exhaust port **172**. Filter **174** may be a bacterial filter to prevent emission of bacteria from housing **120**. A PCB may be provided along with a pressure transducer to control output of the pump in response to pressure measurements calculated in the collection canister.

Collection canister **118** collects exudates "e" removed from the wound bed "w' during therapy through conduit or tubing **106**. A fluid inlet **178** and suction port **180** may be maintained between the housing **120** and the canister **118**. Fluid inlet **178** is configured to operably engage conduit **106**. Fluid inlet **178** may be connectable with conduit **106** by conventional air and fluid tight means, such as those described above. In embodiments, fluid inlet **178** may contain a luer lock or other connector within the purview of those skilled in the art to secure the end of conduit **106** with the fluid inlet **178**. It is envisioned that fluid inlet **178** is configured to receive a cap in order to prevent leakage of exudates and odor from internal chamber **176** of collection canister **118** when housing **120** is separated from the canister **118**.

Suction port **180** is in fluid communication with vacuum source or pump **164**. A filter **182**, such as a hydrophobic membrane or baffling to prevent exudates from being aspirated into pump **164** may be disposed adjacent or within suction port **180.** Filter **182** may also include charcoal or other odor absorbing materials and may prevent the passage of bacteria. Pump **164** creates a vacuum within internal chamber **176** of collection canister **118** by drawing air through suction port **180.**

Collection canister **118** collects the exudates removed from the wound bed "w' during therapy through conduit, or tubing, **106**. Collection canister **118** is releasably connected to housing **120** of control unit **116**. Collection canister **118** may include any container suitable for containing wound fluids. Collection canister **118** may be substantially rigid in order to maintain the integrity and shape of the canister as a stand alone component. In the alternative, collection canister **118** may be relatively flexible and/or partly expandable to accommodate the wound exudates. Collection canister **118** may contain an absorbent material to consolidate or contain the wound drainage or debris. In other embodiments, at least a portion of collection canister **118** may be transparent to assist in evaluating the color, quality, or quantity of wound exudates. This transparency may assist in determining the remaining capacity of the canister or when the canister should be replaced.

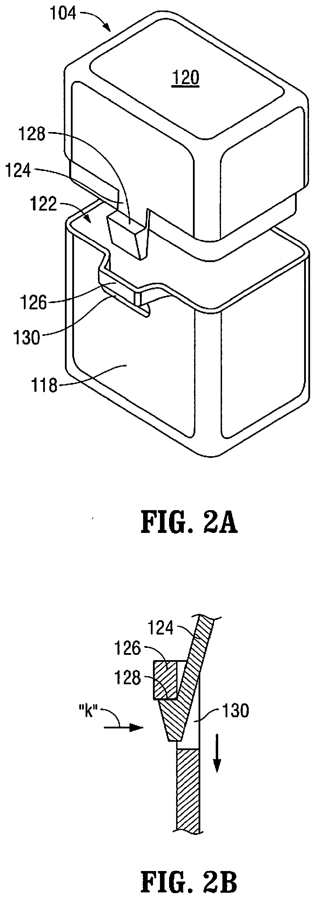

Referring now to **FIG. 2A**, subatmospheric pressure mechanism **104** includes a latch coupling mechanism **122** adapted for selectable releasable coupling of control unit housing **120** and collection canister **118**. Latch coupling mechanism **122** may facilitate the connection, disconnection, or maintenance of components of system **100**, including the replacement of collection canister **118**. Latch coupling mechanism **122** includes first and second coupling segments **124**, **126** associated with control unit housing **120** and collection canister **118**, respectively. First coupling segment **124** may be a latch having locking surface **128**. Locking surface **128** may be a curved or angular portion adapted for releasable engagement with second coupling segment. Second coupling segment **126** includes a notch or locking slot **130** for receiving latch. In the alternative, first coupling segment **124** of control unit housing **120** may be in the form of a locking slot while second coupling segment **126** of collection canister **118** may be a latch.

Latch coupling mechanism **122** is placed in the engaged position through insertion of first coupling segment or latch **124** within locking slot **130** whereby locking surface **128** engages portions of collection canister **118** defining the locking slot **130** in secured relation therewith. Latch coupling mechanism **122** is released by depressing latch **124** inwardly in the direction of directional arrow "k" of **FIG. 2B** toward collection canister **118** such that locking surface **128** is released from within locking slot **130**. Latch **124** has sufficient flexibility to pivot out of engagement with collection canister **118** during movement to the release position. In one embodiment, latch **124** is monolithically formed with control unit housing **120**. Once release is achieved, housing **120** may be lifted from collection canister **118**.

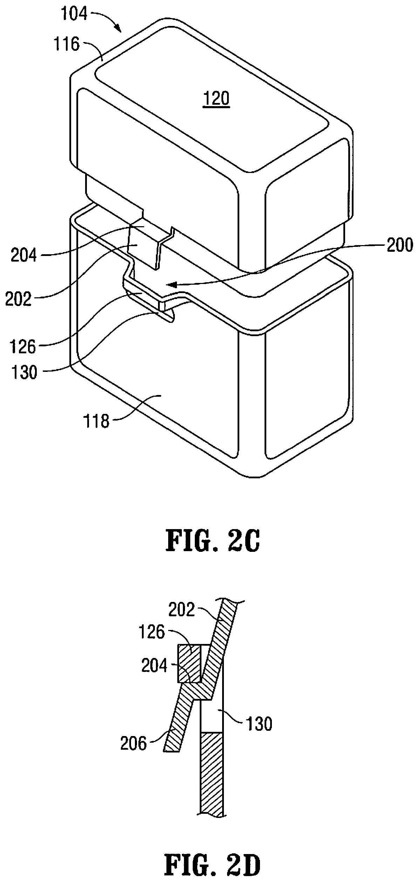

**FIGS. 2C** and **2D** illustrate a latch coupling mechanism according to the claimed invention. Latch coupling mechanism **200** incorporates elongated latch **202** defining a general "z-shape," and having locking surface **204** positioned substantially at the center of the latch **202**. This arrangement of latch **202** provides a manually engaging segment **206** depending from locking surface **204**, which is relatively elongated and displaced from the wall of collection canister **118**. The displaced orientation, in conjunction with the elongated characteristic, may provide mechanical advantages and enhance relative ease of manipulation and/or control of the latch **202** for the clinician. Latch **202** cooperates with locking slot **130** to releasably couple collection canister and control unit housing **120** in a manner similar to the embodiment of **FIGS. 2A-2B****.**

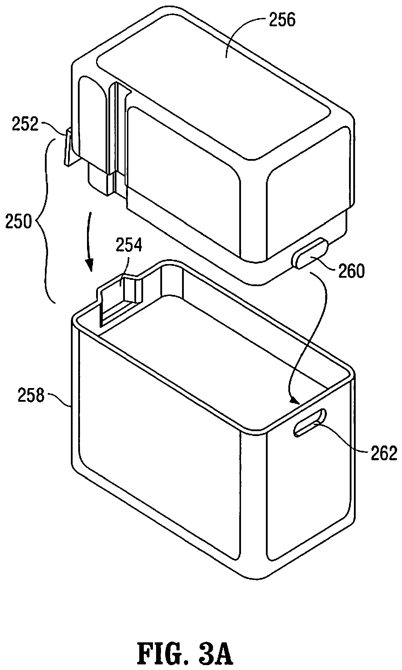

**FIG. 3A** illustrates an alternate example of the latch coupling mechanism of the present disclosure. Latch coupling mechanism **250** includes latch **252** and associated locking slot **254** disposed at respective longitudinal ends of control unit housing **256** and collection canister **258**, respectively. Latch **252** and locking slot **254** function in a manner similar to the latch coupling mechanism of **FIGS. 2A** and **2B**. In addition, control unit housing **256** includes outwardly depending tab **260** adjacent the other longitudinal end of the control unit housing **256**. Tab **260** is received within a corresponding positioned and dimensioned tab slot **262** defined in collection canister **258**. Control unit housing **256** may be mounted to collection canister **258** by pivoting the control unit housing **256** relative to the collection canister **258** in a manner to position tab **260** within tab slot 262. Thereafter, control unit housing **256** is pivoted about the longitudinal end containing tab **260** to insert latch **252** within locking slot **254** until the latch **252** is secured within the locking slot **254**. Release of control unit housing **256** is effected by depressing latch **252** inwardly to be released from locking slot **254**, and, thereafter, pivoting the control unit housing **256** about tab **260** to remove the tab **260** from tab slot **262**.

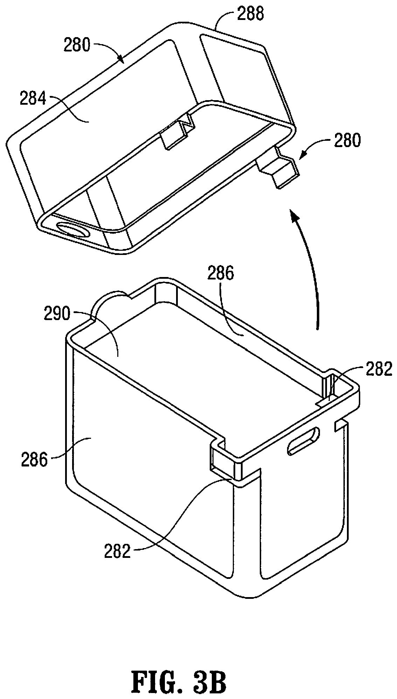

**FIG. 3B** illustrates an alternate example including a pair of latches **280** and associated slots **282** defined within the side walls **284**, **286** of control unit housing **288** and collection canister **290**. In other respects, this example is substantially similar to the example of **FIG. 3A**.

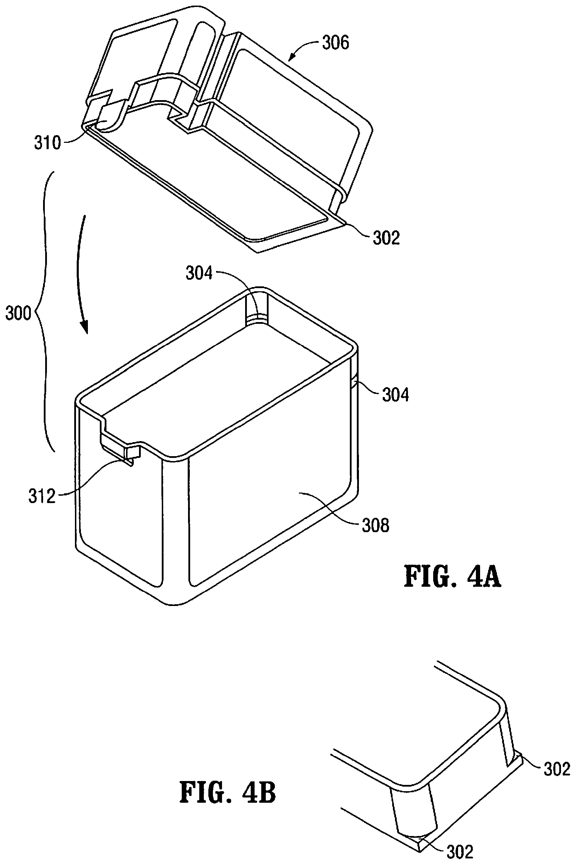

**FIGS. 4A** and **4B** illustrate an alternate example of the latch coupling mechanism of the present disclosure. Latch coupling mechanism **300** incorporates a pair of tabs **302** and associated tab slots **304** at the longitudinal ends of control unit housing **306** and collection canister **308**, respectively. Tabs **302** and slots **304** may be disposed adjacent respective corners to establish a pivot axis for rotating control unit housing **306** relative to collection canister **308** during release and securement of the two components. Latch coupling mechanism **300** further includes locking latch **310** and associated locking slot **312** at the other longitudinal end of control unit housing **306** and collection canister **308**, respectively. Locking latch **310** and locking slot **312** function in a manner similar to the embodiments of **FIGS. 2A-2D**.

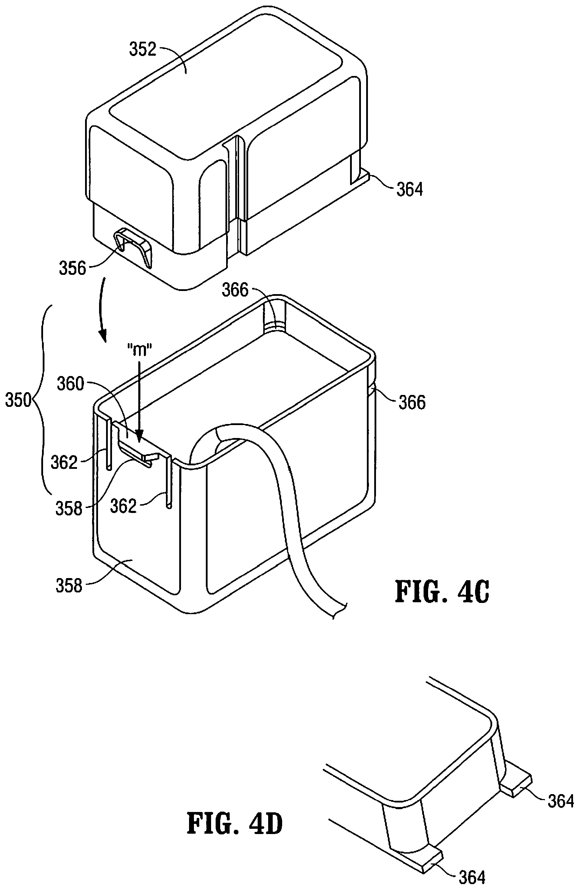

**FIGS. 4C** and **4D** illustrate an alternate example of latch coupling mechanism **350**. Control unit housing **352** and collection canister **354** include latch **356** and locking slot **358**, respectively. Collection canister **354** further includes release tab **360** adjacent locking slot **358** and a pair of relief grooves or slots **362** on each side of the locking slot **358**. Latch **356** is received within locking slot **358** when in the engaged position of collection canister **354** and control unit housing **352**. To release the components, release tab **360** may be depressed in a downward direction "m" indicated in **FIG. 4C** to cause the release tab **360** to pivot outwardly through an angular range of movement and displace locking slot **358** from latch **356**. Such movement of release tab **360** relative to collection canister **354** is facilitated by relief grooves **362**. Upon release of locking slot **358** from latch **356**, control unit housing **352** is removed from collection canister **354**. Latch coupling mechanism may further include a pair of tabs **364** and associated tab slots **366** at the longitudinal ends of control unit housing **352** and collection canister **354**, respectively.

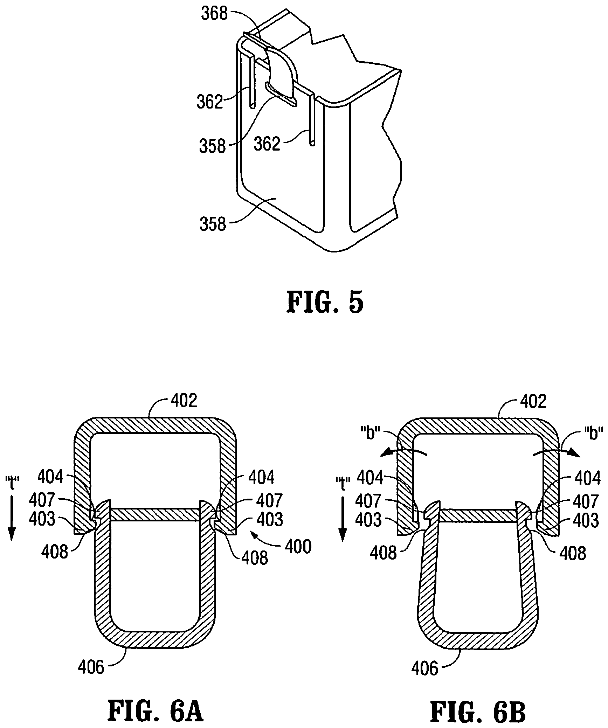

Referring now to **FIG. 5**, an alternate example of latch coupling mechanism **350** incorporates release tongue **368** in lieu of a release tab **360**. Release tongue **368** may be a strip, band, or section of material looped around locking slot **358**. When latch **356** is engaged with locking slot **358**, the operator may pull on tongue **368** in an outward direction thereby deforming portions of collection canister adjacent locking slot **358** as facilitated by relief grooves **362,** to permit release of the latch **356** from the slot **358**, and subsequent release of collection canister **354** from control unit housing **352**.

**FIGS. 6A** and **6B** illustrate an alternate example of latch coupling mechanism **400** in an engaged and disengaged position, respectively. Control unit housing **402** includes a pair of opposed internal locking detents **404** which change position from a locked or contracted state as depicted in **FIG. 6A** to an unlocked or expanded state as depicted in **FIG. 6B** upon external contact. Collection canister **406** incorporates a pair of locking elements **407** associated with a pair of outer locking recesses **408** in opposed relation to the locking detents **404**. Locking elements **407** may be normally biased toward the radial outward position, e.g., in the direction of arrow "b" depicted in **FIG. 6B**. Consequently, as control unit housing 120 is positioned over and depressed or moved in the direction of directional arrow "t" in **FIG. 6A****,** locking detents **404** contact locking elements **407** thereby locking locking detents **404** in a contracted position. With this arrangement, locking elements **407** are directed radially inwardly for reception with control until housing **120**. Once positioned within control until **120**, locking elements **407** are released to permit locking grips **403** to be received within locking recesses **408** to secure collection canister **406** relative to control unit housing **402**.

Collection canister **406** is released from control unit housing **402** by depressing or moving the control unit housing **402** in the direction of directional arrow "t" in **FIG. 6B**. As the control unit housing **402** moves relative to collection canister **406**, locking detents 404 contact locking elements **407** thereby unlocking the locking detents **404** to an expanded state and directing locking elements **407** radially inwardly. Locking grips **403** are now cleared from locking recesses **408**. In this position, control unit housing **402** is free to be removed from collection canister **406** permitting disposal of the canister **406** and subsequent replacement with a new canister.

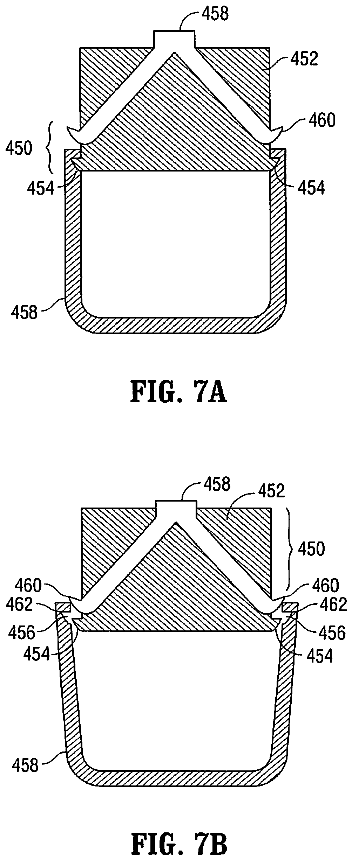

Referring now to **FIGS. 7A** and **7B**, in an alternate example, latch coupling mechanism **450** includes control unit housing **452** having two locking latches **454** disposed in diametrical opposed relation. Locking latches **454** are received within corresponding locking recesses **456** of collection canister **458** when in the engaged or secured position of control unit housing **452** depicted in **FIG. 7A**. Control unit housing **452** further includes release button **458** mounted to the control unit housing **452**. Release button **458** includes outer cam surfaces **460** depending outwardly from control unit housing **452**. Release button **458** is adapted for movement relative to control unit housing **452** from the position depicted in **FIG. 7A** to the position depicted in **FIG. 7B**. During movement of the release button **458** to the position of **FIG. 7B**, outer cam surfaces **460** engage internal surfaces **462** of collection canister **458** and bias the wall surfaces of the canister **458** radially outwardly as shown in **FIG. 7B**. In this position, locking latches **454** are released from locking recesses **456** thereby permitting removal of collection canister **458**. Any means for mounting release button **458** for movement within control unit housing **452** are envisioned.

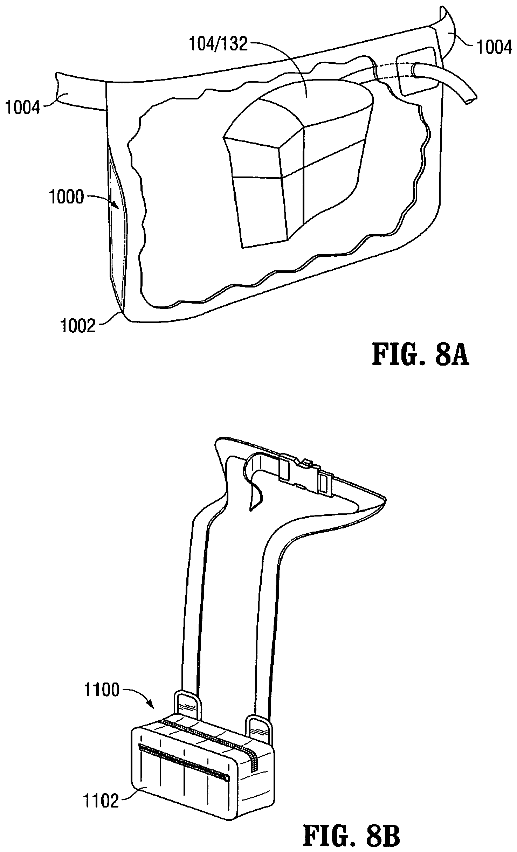

With reference now to **FIG. 8A**, there is illustrated a body support bag **1000** for supporting at least the subatmospheric pressure mechanism **104** and at least canister **118**. As discussed, the wound therapy system **100** of the present disclosure is adapted for mounting to the body of the patient to be a self contained portal unit. In this regard, the subatmospheric pressure mechanism and canister may be at least partially carried or supported by the body support bag **1000**. The body support bag **1000** generally includes a pouch **1002** and at least one strap **1004**, in embodiments, two straps, for securing the pouch **1002** to the body of the patient. The body support bag **1000** is intended to receive and store at least subatmospheric pressure mechanism **104** and collection canister **118**. The body support bag **1000** may be worn about the waist of the patient such as with a belt loop. This is desirable in that it may reduce the length of tubing needed depending on the location of the wound. In addition, the pouch **1002** may be located adjacent the abdomen of the patient which may present a significantly enhanced ability to conceal the system. Tubing **1006** may be secured to the body with tape, straps, or the like, or, optionally, may be unsecured and disposed beneath the patient's clothing. Thus, the body support bag **1000** permits the patient to move without restrictions or limitations, and provides an entirely portable capability to the patient during wound drainage and healing.

**FIG. 8B** illustrates an alternate embodiment of the body support bag. In accordance with this embodiment, the body support bag **1100** is adapted for mounting to the shoulder of the patient and has a pouch **1102**. In other respects, the body support bag **1100** functions in a similar manner to the body support bag of **FIG. 8A**.

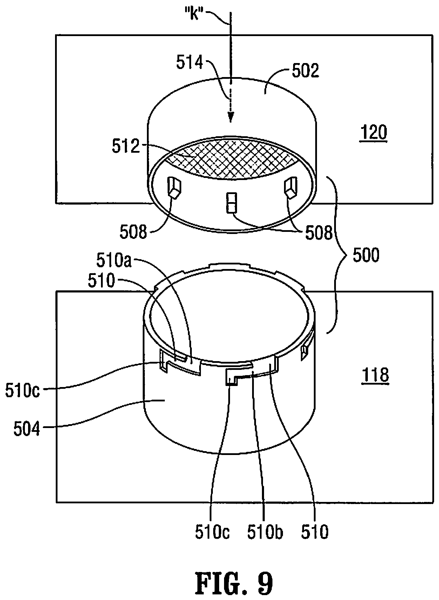

Referring now to **FIG. 9**, an alternate mechanism for coupling control unit housing **120** and collection canister **118** is illustrated. In this example, subatmospheric pressure mechanism **104** includes a bayonet mount or coupling mechanism **500** for selectable releasable coupling and decoupling of control unit housing **120** and collection canister **118**. In this manner, bayonet mount **500** may facilitate the connection, disconnection, or maintenance of components of system **100**, including the replacement of collection canister **118**. In one example, bayonet mount **500** has first and second coupling segments **502, 504** disposed substantially centered on control unit housing **120** and canister **118**, respectively. Coupling segments **502, 504** may be off-centered if desired. First and second coupling segments **502, 504** are generally cylindrical in configuration and depend from their respective component toward each other. First coupling segment **502** may define an internal dimension or diameter slightly greater than a correspondingly internal dimension or diameter of second coupling segment **504** whereby the first coupling segment **502** receives the second coupling segment **504** in the mated condition of the components. In one aspect, either coupling segment **502, 504** may incorporate a gasket or O-ring seal **(not shown)** whereby a substantial seal is formed when the components are coupled. Coupling segment **502** may include at least one internal bayonet projection or bayonet lug **508** depending radially inwardly relative to an axis "k" of the first coupling segment **502**. Second coupling segment **504** includes at least one correspondingly dimensioned and positioned lug retaining slot **510**. In one example, a plurality of bayonet lugs **508** and corresponding retaining slots **510** are radially spaced about their respective components. Each bayonet lug **508** may be a pin, knob, ball, knot, or other protrusion transverse to longitudinal axis "k."

Lug retaining slot **510** is a generally "L", "U" or "Z" shaped recess, through-hole, groove, or the like. For example, each lug retaining slot **510** has lug receiving segment **510a**, transverse segment **510b** and retention segment **510c**. Bayonet mount **500** further includes a spring mechanism or resilient member **512** disposed at the intersection of first and second coupling segments **502, 504** when mated. Spring mechanism **512** may be in the form of a resilient or elastomeric washer or disc disposed within first coupling segment **502**. Spring mechanism **512** facilitates retention of bayonet lugs **508** within retaining slots **510** by biasing the first coupling segment **502** away from the second coupling segment **504** when the coupling segments **502, 504** are mated as will be discussed.

First and second coupling segments **502, 504** also may include visual indicia **514** to facilitate alignment of the respective bayonet lugs **508** and retaining slots **510** of the first and second coupling segments **502, 504**. Such visual indicia may be arrows, dots, lines on the exterior surfaces of first and second coupling segments **502, 504** as depicted in **FIG. 9****.**

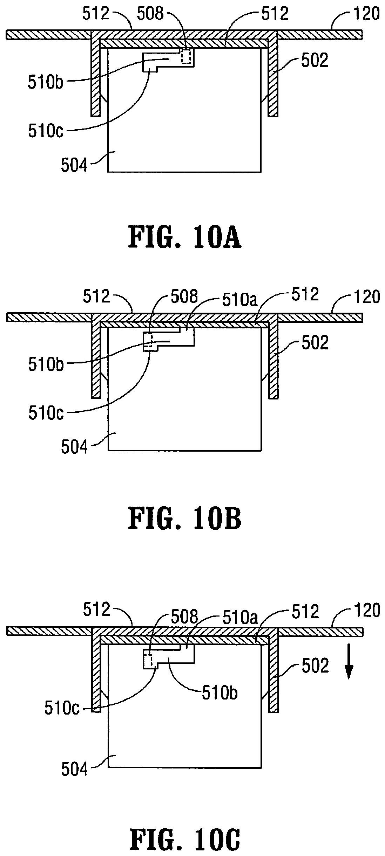

As illustrated in **FIGS. 10A-10C**, first coupling segment **502** is positioned over second coupling segment **504** when control unit housing **120** is aligned with canister **118**. Each bayonet lug **508** enters lug receiving segment **510a** of each retaining slot **510** and is advanced to be aligned with transverse segment **510b** of the lug retaining slot **510**. (**FIG. 10A**). During this movement, spring mechanism **512** is compressed as depicted in **FIG. 10B**. Thereafter, first and second coupling segments **502, 504** are rotated relative to each other through a predetermined angular sector of rotation whereby bayonet lug 508 is adjacent retention segment **510c**. The clinician then may release at least one of the coupling segments **502, 504**, which, effectively causes each bayonet lug **508** to be received within retention segment **510c** of retaining slot **510** under the bias of spring mechanism **512**. Spring mechanism **512** possesses sufficient resiliency to effectively secure bayonet lug(s) **508** within its respective retention segment(s) **510c** of retaining slot(s) **510**. **FIG. 10C** illustrates spring mechanism **512** expanded to secure bayonet lug **508** within retention segment **510c**.

Removal of collection canister **118** from control unit housing **120** may be performed by advancing collection canister **118** relative to control unit housing **120** against the bias of spring mechanism **512** whereby each bayonet lug **508** traverses retention segment **510c** to be in alignment with transverse segment **510b**. Collection canister **118** and control unit housing **120** are rotated relative to each other to cause each bayonet lug **508** to be positioned in alignment with a corresponding lug receiving segment **510a** of retaining slot **510**. Collection canister **118** is then removed from control unit housing **120** with bayonet lug(s) **508** exiting the lug receiving segments **510a.**

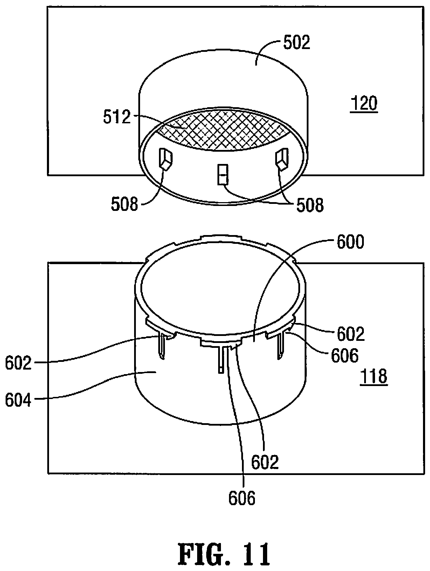

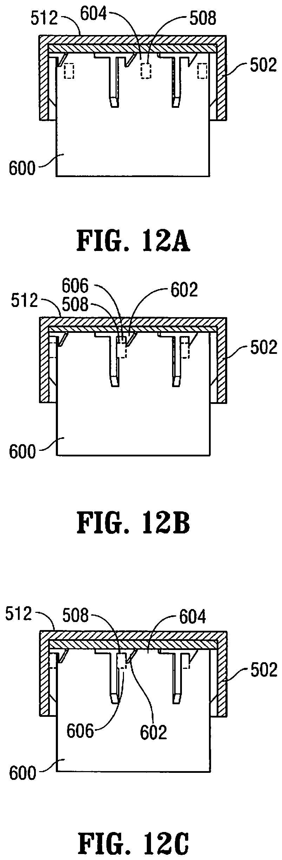

**FIGS. 11** and **12A****-12C** illustrate an alternate example of a bayonet coupling. In accordance with this example, second coupling segment **600** incorporates a plurality of latches **602** to secure bayonet lugs **508**. In use, bayonet lugs **508** are aligned with recesses **604** of second coupling segment **600** to position first coupling segment **502** relative to second coupling segment **600** (**FIG. 12A**). Thereafter, first and second coupling segments **502, 600** are rotated relative to each other whereby bayonet lugs pass **508** pass latches **602** to be received within locking recesses **606** (**FIG. 12B**). Latches **602** are sufficiently flexible to permit passage of bayonet lugs **508**, but, return to their original position once received within locking recesses **606** (**FIG. 12C**). This mechanism may provide a more permanent connection. As an alternative, first coupling segment **502** may incorporate spring mechanism **512** which may be compressed to displace bayonet lugs **508** from locking recesses **606** thereby permitting relative rotational movement of the first and second coupling segments **502, 600** and removal of collection canister **118** from control unit housing **120**.

Bayonet mount **500** may be constructed as shown in the exemplary examples above, or in reverse so that coupling segment **502** is on canister **118** and coupling segment **504, 600** is on control unit housing **120**. Further, the number, length, angle, and size of bayonet lug(s) **508** of coupling segment **502** may vary and accordingly, retaining slot **510** of coupling segment **504** will correspond to the size, number, and configuration of projection(s) **508** so that control unit housing **120** and canister **118** may be releasably joined together.

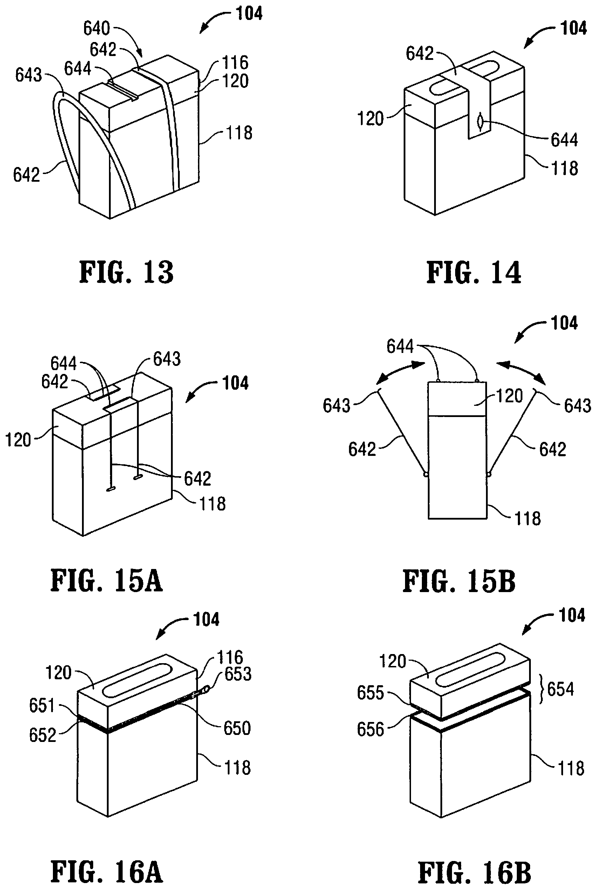

Referring now to **FIG. 13** another example of releasably connecting collection canister **118** and control unit housing **120** of subatmospheric pressure mechanism **104** is illustrated. Fastener mechanism **640** includes strap **642** and catch **644**. Strap **642** may be of a predetermined length sufficient to wrap around housing **120** and firmly hold housing **120** to canister **118**. Strap **642** may also be made of material having an elastic component in order for strap **642** to stretch around housing **120** and join the two components. In the alternative, strap **642** may be substantially rigid and formed of steel, titanium, a polymeric material or the like. Strap **642** includes closed looped end **643**. Looped end **643** is passed around canister **118** and housing **120** to securely and firmly connect housing **120** to canister **118**. Catch **644** are used to secure strap **642** to housing **120**. In the current example, catch **644** is in the form of indentations substantially parallel to the path of strap **642** for holding strap **642** on housing **120**. To release, the operator pulls strap **642** away from housing **120**. In examples, strap **642** has sufficient elasticity that it may catch on housing **120** without the use of catch **644**.

**FIG. 14** illustrates another example of canister **118** having strap **642** and catch **644**. Strap **642** is affixed to a side of canister **118** and catch **644** is disposed on an opposite side of canister **118**. Catch **644** may be a button, knob, hook, clasp, bar, or other protrusion capable of securing strap **642**. In examples, catch **644** may be one half of a fixation element such as a hook and loop fastener. To join housing **120** and canister **118**, the operator passes strap **642** over housing **120** and secures it with catch **644**.

**FIGS. 15A** and **15B** illustrate another example of canister **118** having an alternative placement of straps **642** and an alternate catch **644** than **FIGS. 13** and **14****.** Canister **118** includes strap **642** anchored on canister **118** to form closed looped end **643**. Housing **120** includes catch **644** in the form of a bar, but other protrusions as described above may be used. Strap **642** is placed around catch **644** thus preventing strap **642** from moving and releasably connecting canister **118** with housing **120**. The operator may then release housing **120** by freeing strap **642** from catch **644**.

In other examples, the fastener mechanism may include complementary joining members disposed around the periphery of housing **120** and canister **118**, examples of which are shown in **FIGS. 16-21****.** **FIG. 16A** illustrates housing **120** of control unit **116** and collection canister **118** releasably connected by zipper **650**. Zipper **650** includes first teeth assembly **651**, second teeth assembly **652**, and slider **653**. First and second teeth assemblies **651** and **652** are disposed on housing **120** and canister **118**, respectively. Slider **653** may be attached to either first or second teeth assemblies **651** or **652**. As known to those skilled in the art, slider **653** includes wedges that combine the hooks and hollows of first and second teeth assemblies **651** and **652** in order to fasten first and second teeth assemblies **651** and **652** together. **FIG. 16B** illustrates use of hook and loop fastener **654**. Fastener **654** includes hook **655** and loop **656** material, each disposed on one of housing **120** and canister **118**. As housing **120** is brought in contact with canister **118**, the pressure exerted causes hooks **655** to entangle loops **656** to form a bond strong enough to hold housing **120** and canister **118** together. By exerting pressure to separate housing **120** from canister **118**, hook and loop bonds are broken a few at a time along the length of fastener **654** in order to remove housing **120** from canister **118**.

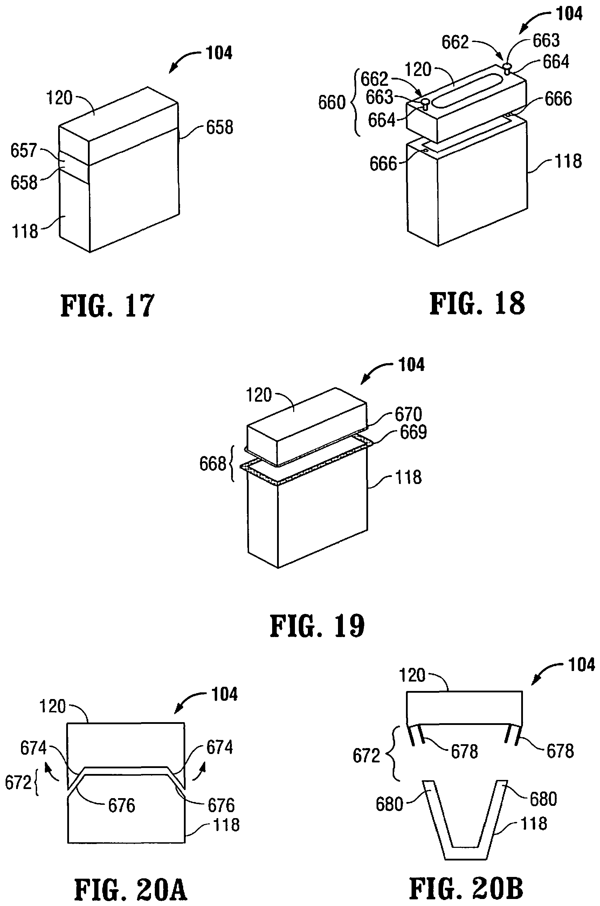

**FIG. 17** illustrates use of door fastener **658**. Door fastener **658** includes door **657**. Door **657** is unfastened so that housing **120** may be placed on top of canister **118**. Once placed together, door **657** is closed and locked in place. In examples, door **657** is located on canister **118**. In other examples, door **657** is located on housing **120**. It is envisioned that more than one door **657** may be used to securely engage canister **118** with housing **120**.

**FIG. 18** illustrates use of screw fastener **660**. Screw fastener **660** includes thumbscrews **662** through housing **120** and receiving slots **666** in canister **118**. Thumbscrew **662** includes gripping head **663** and threaded body **664**. Head **663** may include ridged sides **665** for engaging the operator's fingers for better control of thumbscrew **662**. The operator twists head **663** of thumbscrew **662** until threaded body **664** is firmly interlocked with slot **666** of canister **118**. To release, thumbscrew **662** is twisted in the opposite direction. In examples, head **663** of thumbscrew **662** may be slotted for receiving a screwdriver.

**FIG. 19** illustrates use of ring fastener **668**. Ring **669** is disposed around canister **118** and gasket **670** on housing **120**. Ring **669** may be fixed at one end to canister **118** or may be independently associated with canister **118**. As housing **120** is placed on canister **118** gasket **670** deforms and compresses. Ring **669** may then be slidingly engaged around both housing **120** and canister **118** utilizing gasket **670** as a securement point. In examples, ring fastener **668** is constructed in reverse so that sliding ring **669** is disposed on housing **120** and gasket **670** on canister **118**.

**FIGS. 20A** and **20B** illustrate use of friction fit fastener **672**. **FIG. 20A** illustrates housing **120** having angled edges **674** and canister **118** having complementary angled edges **676**. As housing **120** is placed on canister **118** angled edges **674** of housing **120** slide over angled edges **676** of canister **118** slightly displacing edges **674** causing edges **674** to grip edges **676**, thus holding housing **120** and canister **118** together. In examples, a gasket may be used between housing **120** and canister **118** to aid in removably sealing the two components together. **FIG. 20B** utilizes groove **678** in housing **120** and tongue **680** on canister **118**. Tongue **680** is sized comparably to groove **678** so that as tongue **680** is inserted into groove **678** they frictionally fit together thus securing housing **120** with canister **118**. In examples, friction fit fastener **672** is constructed in reverse, such that tongue **680** is located on housing **120** and groove **678** in canister **118**.

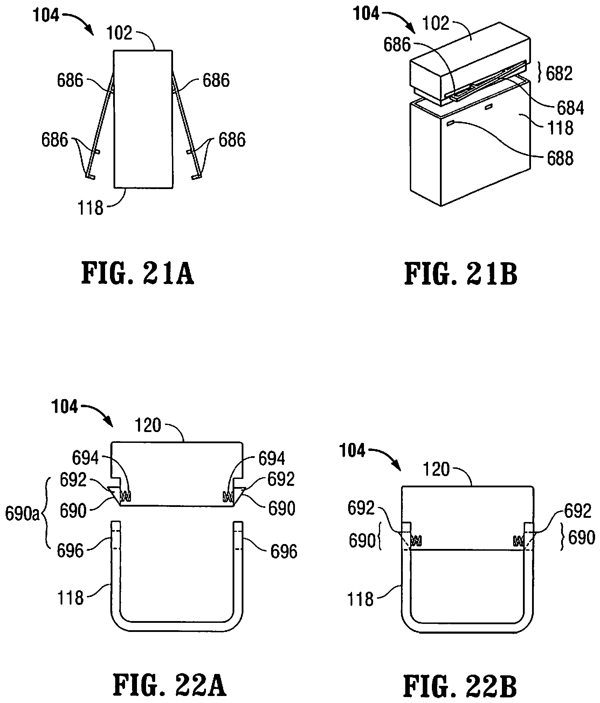

**FIGS. 21A** and **21D** illustrate use of snap fastener **682** to facilitate connection between housing **120** and canister **118**. Snap fastener **682** includes bar **684** having protrusions **686**. One end of bar **684** is pivotably connected to housing **120**. Indentations **688** are positioned on canister **118** such that when housing **120** is positioned on canister **118** and bar **684** pivoted towards canister **118**, protrusions **686** and indentations **688** align and snap fit together thus joining canister **118** and housing **120**. To release, the operator pulls bar **684** away from canister **118** thus disengaging protrusions **686** from indentations **688**. In examples, snap fastener **682** is constructed in reverse, such that indentations **688** are located on housing **120** and bar **684** on canister **118**.

In examples, the fastener mechanism comprises spring-loaded button fastener **690**. Examples are included in **FIGS. 22-24**. In **FIGS. 22A** and **22B**, housing **120** includes spring-loaded buttons **690**. Button **690** includes button **692** and spring **694**. Spring-loaded button **690a** is shown in its unbiased state, while button **690b** is shown in a biased position. Button **692** generally lies at an angle with the top portion protruding farther out from housing **120** than the bottom portion so that as housing **120** slides onto canister **118**, buttons **692** compress against canister **118**, returning to their biased position when reaching opening **696** in canister **118**. The slope of button **692** also prevents housing **120** from slidingly detaching from canister **118** unless the operator presses button **692** in towards canister **118**. In examples, buttons **692** are placed on the longer sides of canister **118** close to one end so that the operator may press both buttons **692** with one hand.

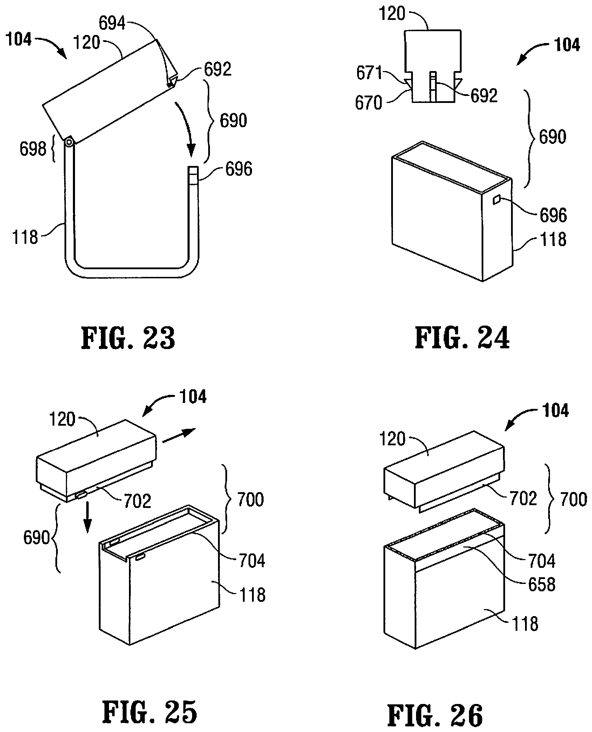

**FIG. 23** illustrates another example for fastening housing **120** and canister **118** using spring-loaded button fastener **690** and pivot point **698**. Spring-loaded button fastener **690** is placed on one end of housing **120** and the other end is permanently or removably coupled to canister **118** at pivot point **698**. The operator pivots housing **120** down towards canister **118** to engage spring-loaded button fastener **690**. To disengage, the operator presses button **692** and pivots housing **120** back up. In examples, subatmospheric pressure mechanism **104** is a disposable unit. In other examples, canister **118** can be used with a disposable collection bag **(not shown)** placed therein. **FIG. 24** illustrates another example using spring-loaded button fastener **690**. Gasket **670** is also utilized and includes grooved surface **671** which deforms and sticks down to canister **118** when housing **120** is placed on canister **118** and spring-loaded button fastener **690** is engaged.

In yet other examples, as illustrated in **FIGS. 25** and **26**, the fastener mechanism includes sliding mechanism **700**. Sliding mechanism includes rail **702** and track **704**, one of each being disposed on housing **120** and canister **118**. Housing **120** and canister **118** are engaged by placing one end of housing **120** adjacent one end of canister **118** such that rail **702** and track **704** align. Housing **120** may then slide across canister **118** to a closed position. An additional fastening mechanism may be used in conjunction with mechanism **700**, such as spring-loaded buttons **690** as illustrated in **FIG. 25** and door **658** in **FIG. 26**.

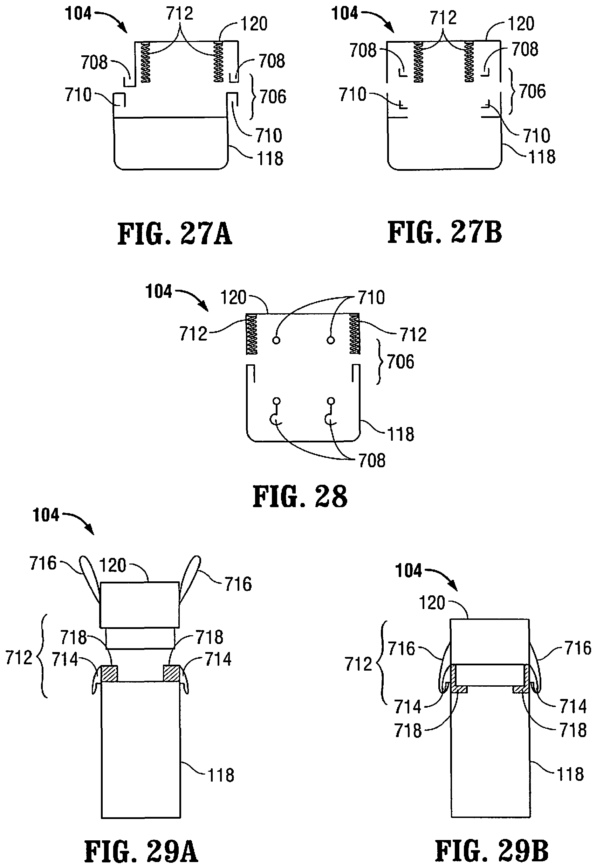

In other examples, the fastener mechanism may include spring-assisted clips **706**. Spring-assisted clips **706** include hook element **708**, grab element **710**, and springs **712**. **FIGS. 27A** and **27B** illustrate alternate examples utilizing clips **706**. Hook element **708** is disposed on housing **120** and springs **712** are placed adjacent to each hook element **708**. Grab element **710** is located on canister **118** and is complementary to hook element **708**. Housing **120** is placed on top of canister **118** and pressed down, so that springs **712** compress against canister **118**. Housing **120** is slid onto canister **118** so that hook element **708** catches grab element **710**. As the operator releases pressure on housing **120**, springs **712** spring up and housing **120** remains connected to canister **118**. To disconnect, the operator presses back down on housing **120** so that hook and grab elements **708** and **710** disconnect. The operator then slides housing **120** away from canister **118**. Other examples, such as **FIG. 28**, illustrate alternate placement and types of hook and grab elements **708** and **710**, respectively.

**FIGS. 29A** and **29B** illustrate use of movable clip fastener **712** to releasably join housing **120** with canister **118**. Clip fastener **712** includes fixed grab element **714**, movable hook element **716**, and gasket **718**. Housing **120** includes movable hook element **716** and canister **118** includes fixed grab element **714** and gasket **718**. As housing **120** is placed on canister **118**, gasket **718** deforms and seals canister **118**. Movable hook elements **716** are brought down towards canister **118** and hooked on fixed grab elements **714** thus connecting housing **120** and canister **118**. To release, the operator releases movable hook element **716** from fixed grab element **714**. The operator may also first press down on housing **120** to compress gasket **718** thus creating room to release movable hook element **716** from fixed grab element **714**.

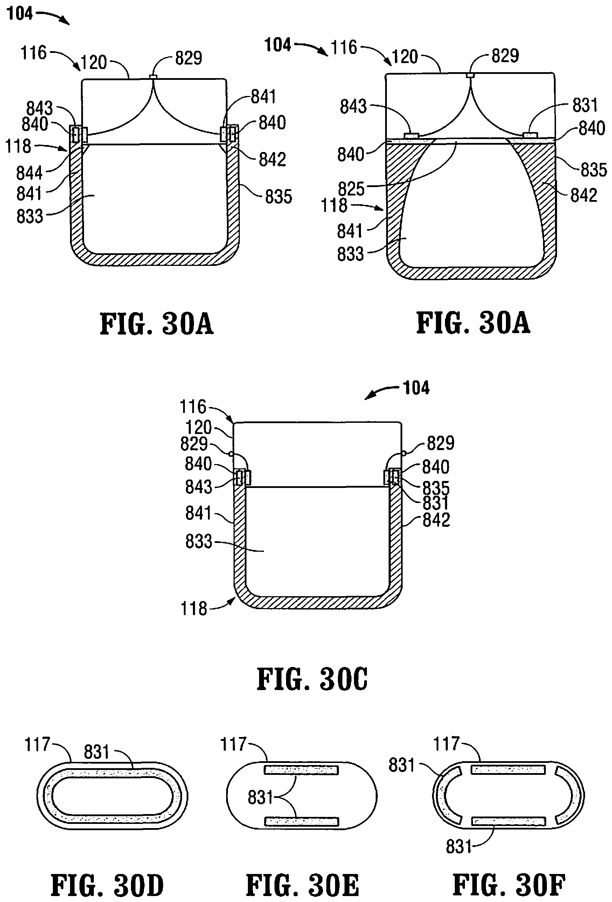

Referring now to **FIGS. 30A-30C**, other examples of releasably connecting collection canister **118** and control unit housing **120** are illustrated. Housing **120** includes switch **829** and at least one electromagnet **831** disposed along the periphery of housing **120**. Switch **829** may be a button, knob, dial, or other adjuster capable of activating and/or de-activating electromagnet **831** by way of control circuitry **(not shown).** Switch **829** may be manually actuated, or, in the alternative, may be automatically actuated upon mounting of some of the components of the subatmospheric pressure mechanisms **104**. The control circuitry recognizes changes in state of switch **829** and activates or de-activates the magnetic field of electromagnet **831** by altering the flow of current from a power source **168 (not shown).** The magnetic field of electromagnet **831** can be manipulated over a wide range by controlling the amount of electric current from the power source.

Collection canister **118** includes magnetic or ferromagnetic material which may be disposed within or adjacent to upper portion **835** of canister **118.** Magnetic material **840** may be a permanent magnet or metal capable of being attracted by the magnetic field created by electromagnet **831** of housing **120** of control unit **116.** Such materials include nickel, iron, cobalt, their alloys, and the like. Other materials, within the purview of those skilled in the art, containing detectable magnetic properties may be used. Magnetic material **840** may be placed anywhere on canister **118.** For example, **FIG. 30A** shows magnet material **840** in the form of an annular magnet disposed within the side walls **841** and **842** of canister **118.** **FIG. 30B** shows magnetic material **840** disposed on top wall **843.** **FIG. 30C** shows upper portion **835** of canister **118** made of magnetic material **840** and thus, a portion of canister **118** itself is magnet **840**.

Housing **120** of control unit **116** is releasably connected to collection canister **118.** Housing **120** of control unit **116** may be placed partially within canister **118** such as shown in **FIG. 30A**. Control unit housing **120** is supported by supports **844** at least partially disposed within canister **118**. Control unit housing **120** may also be placed on top of canister **118** as shown in **FIG. 30B**. **FIG. 30B** also contains a lip or seal **825** to aid in aligning housing **120** of control unit **116** on canister **118** and to tightly close or seal canister **118**.

When housing **120** of control unit **116** and canister **118** are mounted to each other, electromagnet **831** of control unit housing **120** and magnetic material **840** of canister **118** are substantially aligned or positioned such that magnets **831** and **840** are capable of attracting each other. Switch **829** may be indented within housing **120** to prevent inadvertent activation. In examples, such as **FIG. 30C**, two switches **829** are utilized thus making it more difficult to mistakenly engage both switches **829** and disconnect the components. Any number of magnets may be disposed around housing **120** of control unit **116** and controlled by switch **829**, such as the configurations shown in **FIGS. 30D-30F**. As indicated hereinabove, switch **829** may be manually activated and may, e.g., be mounted to the exterior of either housing **120** or canister **118**.

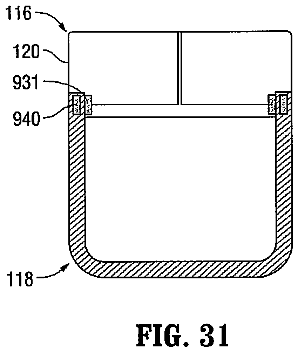

Alternatively, **FIG. 31** illustrates use of a permanent magnet **931** instead of an electromagnet in control unit housing **120**. The distance created between magnets **931** and **940** or change in dipole moments breaks the magnetic field and frees control unit housing **120** from canister **118**.

To assemble the control unit housing **120** and the canister **118**, the housing **120** and canister **118** are aligned and joined together by activating the magnetic field of electromagnet **831** by switch **829** of **FIGS. 30A-30C** (or the magnetic field of permanent magnets **931** and **940** of **FIG. 31**). Alternatively, housing **120** of control unit **116** and canister **118** may have complimentary sensors for detecting attachment which in turn signal control circuitry to activate a power source. Control unit housing **120** and canister **118** remain coupled until switch **829** is triggered and electromagnet **831** de-activated.

## Claims

1. A portable system (100) for subatmospheric pressure therapy in connection with healing a surgical wound, which comprises: a wound dressing (102) dimensioned for positioning relative to a wound bed (w) of a subject; and a subatmospheric pressure mechanism (104) dimensioned to be carried or worn by the subject, the subatmospheric pressure mechanism (104) including a housing (120) having a control unit, a collection canister (118) in fluid communication with the wound dressing (102) for collecting exudates from the wound bed (w) removed under subatmospheric pressure supplied by the control unit, and means for releasably connecting the housing (120) and the canister (118) **characterized in that** the means for releasably connecting the housing (120) and the canister (118) includes a latch coupling mechanism (200) that incorporates an elongated latch (202) defining a general "z-shape" and comprises a locking surface (204) positioned substantially at the center of the latch (202).

2. The system of claim 1 wherein a manually engaging segment depends from the locking surface wherein said manually engaging segment is relatively elongated and displaced from a wall of the collection canister.

3. The system of claim 1 or claim 2, wherein the latch coupling mechanism includes a first coupling segment and a second coupling segment, the first coupling segment including a locking surface and the second coupling segment including a locking slot for securely receiving the locking surface of the first coupling segment.

4. The system of claim 3, wherein the latch is configured to cooperate with the locking slot to releasably couple the collection canister and the housing.

5. The system of claim 3 or claim 4, wherein the first coupling segment is associated with the housing and the second coupling segment is associated with the collection canister.

6. The system of any of claims 3 to 5, wherein the locking surface is an angular portion adapted for releasable engagement with the second coupling segment.

7. The system of any preceding claim, wherein the housing houses the software, logic and components required to operate the subatmospheric pressure mechanism.

8. The system of any preceding claim, wherein the subatmospheric pressure mechanism incorporates a vacuum source or pump, an actuator or motor for activating the vacuum source and a power source.

9. The system of claim 8, wherein the housing further includes a vent portal configured to exhaust air from the vacuum source or pump through an exhaust port.

10. The system of claim 8 or claim 9, wherein a suction port is in fluid communication with the vacuum source or pump and wherein the pump is configured to create a vacuum within an internal chamber of the collection canister by drawing air through the suction port.

11. The system of claim 10 comprising a filter disposed adjacent or within the suction port wherein said filter is configured to prevent exudates from being aspirated into said pump.

12. The system of any preceding claim, wherein at least a portion of the collection canister is transparent to assist in evaluating the colour, quality or quantity of wound exudates.

13. The system of any preceding claim comprising a conduit extending from subatmospheric pressure mechanism configured to provide fluid communication between the interior of the wound dressing and subatmospheric pressure mechanism.

14. The system of any preceding claim, further comprising a body support bag for supporting at least the subatmospheric pressure mechanism and at least the collection canister.

15. The system of claim 14, wherein the body support bag includes a pouch and at least one strap for securing the pouch to the body of a patient and wherein the body support bag is intended to receive and store at least the subatmospheric pressure mechanism and the collection canister.

## Patentansprüche

1. Ein tragbares System (100) für Unterdrucktherapie im Zusammenhang mit der Heilung einer Operationswunde, das Folgendes beinhaltet: eine Wundauflage (102), die dazu bemessen ist, relativ zu einem Wundbett (w) einer Person positioniert zu werden; und einen Unterdruckmechanismus (104), der dazu bemessen ist, von der Person getragen oder angezogen zu werden, wobei der Unterdruckmechanismus (104) Folgendes umfasst: ein Gehäuse (120) mit einer Steuereinheit, einen Sammelbehälter (118) in Fluidverbindung mit der Wundauflage (102) zum Sammeln von Exsudaten aus dem Wundbett (w), die unter von der Steuereinheit geliefertem Unterdruck abgeführt werden, und Mittel zum lösbaren Verbinden des Gehäuses (120) und des Behälters (118), **dadurch gekennzeichnet, dass** das Mittel zum lösbaren Verbinden des Gehäuses (120) und des Behälters (118) einen Rastenkupplungsmechanismus (200) umfasst, der eine längliche Raste (202) einschließt, die eine allgemeine "z-Form" definiert und eine Arretierfläche (204) beinhaltet, die im Wesentlichen in der Mitte der Raste (202) positioniert ist.

2. System gemäß Anspruch 1, wobei ein handbetätigtes Eingriffssegment von der Arretierfläche ausgeht, wobei das handbetätigte Eingriffssegment relativ länglich und von einer Wand des Sammelbehälters versetzt ist.

3. System gemäß Anspruch 1 oder Anspruch 2, wobei der Rastenkopplungsmechanismus ein erstes Kupplungssegment und ein zweites Kupplungssegment umfasst, wobei das erste Kupplungssegment eine Arretierfläche umfasst und das zweite Kupplungssegment einen Arretierschlitz umfasst, um die Arretierfläche des ersten Kupplungssegments sicher aufzunehmen.

4. System gemäß Anspruch 3, wobei die Raste konfiguriert ist, um mit dem Arretierschlitz zusammenzuwirken, um den Sammelbehälter und das Gehäuse lösbar zu koppeln.

5. System gemäß Anspruch 3 oder Anspruch 4, wobei das erste Kupplungssegment dem Gehäuse zugeordnet ist und das zweite Kupplungssegment dem Sammelbehälter zugeordnet ist.

6. System gemäß Ansprüchen 3 bis 5, wobei die Arretierfläche ein gewinkelter Abschnitt ist, der für lösbaren Eingriff mit dem zweiten Kupplungssegment ausgelegt ist.

7. System gemäß einem der vorhergehenden Ansprüche, wobei das Gehäuse die Software, Logik und Komponenten beherbergt, die für den Betrieb des Unterdruckmechanismus erforderlich sind.

8. System gemäß einem der vorhergehenden Ansprüche, wobei der Unterdruckmechanismus eine Vakuumquelle oder Pumpe, einen Aktuator oder Motor zur Aktivierung der Vakuumquelle und eine Stromquelle einschließt.

9. System gemäß Anspruch 8, wobei das Gehäuse ferner ein Entlüftungsportal umfasst, das konfiguriert ist, um Luft von der Vakuumquelle oder Pumpe durch eine Auslassöffnung auszulassen.

10. System gemäß Anspruch 8 oder Anspruch 9, wobei eine Saugöffnung in Fluidverbindung mit der Vakuumquelle oder Pumpe steht und wobei die Pumpe konfiguriert ist, um durch Ziehen von Luft durch die Saugöffnung ein Vakuum innerhalb einer Innenkammer des Sammelbehälters zu erzeugen.

11. System gemäß Anspruch 10, das einen Filter beinhaltet, der neben oder innerhalb der Saugöffnung angeordnet ist, wobei der Filter konfiguriert ist, um zu verhindern, dass Exsudate in die Pumpe aspiriert werden.

12. System gemäß einem der vorhergehenden Ansprüche, wobei mindestens ein Abschnitt des Sammelbehälters transparent ist, um die Bewertung der Farbe, Qualität oder Menge der Wundexsudate zu unterstützen.

13. System gemäß einem der vorhergehenden Ansprüche, das eine Leitung beinhaltet, die sich von dem Unterdruckmechanismus erstreckt und konfiguriert ist, um eine Fluidverbindung zwischen dem Inneren der Wundauflage und dem Unterdruchmechanismus bereitzustellen.

14. System gemäß einem der vorhergehenden Ansprüche, das ferner eine Körpertragetasche beinhaltet, um mindestens den Unterdruckmechanismus und mindestens den Sammelbehälter zu tragen.

15. System gemäß Anspruch 14, wobei die Körpertragetasche einen Beutel und mindestens einen Gurt umfasst, um den Beutel an dem Köper eines Patienten zu befestigen, und wobei die Körpertragetasche dazu dient, mindestens den Unterdruckmechanismus und den Sammelbehälter aufzunehmen und aufzubewahren.

## Revendications

1. Un système portable (100) pour thérapie par pression subatmosphérique en lien avec la cicatrisation d'une plaie chirurgicale, qui comprend : un pansement pour plaie (102) dimensionné pour être positionné par rapport à un lit de plaie (w) d'un sujet ; et un mécanisme de pression subatmosphérique (104) dimensionné afin d'être transporté ou porté par le sujet, le mécanisme de pression subatmosphérique (104) incluant un logement (120) ayant une unité de commande, un récipient de collecte (118) en communication fluidique avec le pansement pour plaie (102) pour collecter des exsudats du lit de plaie (w) retirés sous pression subatmosphérique apportée par l'unité de commande, et un moyen pour raccorder de manière amovible le logement (120) et le récipient (118) **caractérisé en ce que** le moyen pour raccorder de manière amovible le logement (120) et le récipient (118) inclut un mécanisme de couplage à loquet (200) qui incorpore un loquet allongé (202) définissant une « forme en z » générale et comprend une surface de verrouillage (204) positionnée substantiellement au centre du loquet (202).

2. Le système de la revendication 1 dans lequel un segment se mettant en prise manuellement dépend de la surface de verrouillage, ledit segment se mettant en prise manuellement étant relativement allongé et décalé d'une paroi du récipient de collecte.

3. Le système de la revendication 1 ou de la revendication 2, dans lequel le mécanisme de couplage par loquet inclut un premier segment de couplage et un deuxième segment de couplage, le premier segment de couplage incluant une surface de verrouillage et le deuxième segment de couplage incluant une fente de verrouillage pour recevoir de manière sûre la surface de verrouillage du premier segment de couplage.

4. Le système de la revendication 3, dans lequel le loquet est configuré afin de coopérer avec la fente de verrouillage afin de coupler de manière amovible le récipient de collecte et le logement.

5. Le système de la revendication 3 ou de la revendication 4, dans lequel le premier segment de couplage est associé au logement et le deuxième segment de couplage est associé au récipient de collecte.

6. Le système de n'importe lesquelles des revendications 3 à 5, dans lequel la surface de verrouillage est une partie angulaire conçue pour une mise en prise amovible avec le deuxième segment de couplage.

7. Le système de n'importe quelle revendication précédente, dans lequel le logement abrite le logiciel, la logique et les composants nécessaires pour faire fonctionner le mécanisme de pression subatmosphérique

8. Le système de n'importe quelle revendication précédente, dans lequel le mécanisme de pression subatmosphérique incorpore une source de vide ou une pompe, un actionneur ou un moteur pour activer la source de vide et une source d'alimentation.

9. Le système de la revendication 8, dans lequel le logement inclut en outre un portail d'évent configuré afin d'évacuer de l'air de la source de vide ou de la pompe par un orifice d'évacuation.

10. Le système de la revendication 8 ou de la revendication 9, dans lequel un orifice d'aspiration est en communication fluidique avec la source de vide ou la pompe et dans lequel la pompe est configurée afin de créer un vide au sein d'une chambre interne du récipient de collecte en aspirant de l'air par l'orifice d'aspiration.

11. Le système de la revendication 10 comprenant un filtre disposé de façon adjacente à ou au sein de l'orifice d'aspiration, ledit filtre étant configuré afin d'empêcher les exsudats d'être aspirés dans ladite pompe.

12. Le système de n'importe quelle revendication précédente, dans lequel au moins une partie du récipient de collecte est transparente afin de faciliter l'évaluation de la couleur, de la qualité ou de la quantité des exsudats de plaie.

13. Le système de n'importe quelle revendication précédente comprenant un conduit s'étendant à partir d'un mécanisme de pression subatmosphérique configuré afin de fournir une communication fluidique entre l'intérieur du pansement pour plaie et le mécanisme de pression subatmosphérique.

14. Le système de n'importe quelle revendication précédente, comprenant en outre un sac de soutien corporel pour soutenir au moins le mécanisme de pression subatmosphérique et au moins le récipient de collecte.

15. Le système de la revendication 14, dans lequel le sac de soutien corporel inclut une poche et au moins une sangle pour fixer la poche sur le corps d'un patient et dans lequel le sac de soutien corporel est destiné à recevoir et à stocker au moins le mécanisme de pression subatmosphérique et le récipient de collecte.
